(19) Europäisches Patentamt
European Patent Office
Office européen des brevets

(11) **EP 3 424 450 B1**

(12) **EUROPEAN PATENT SPECIFICATION**

(45) Date of publication and mention
of the grant of the patent:
**17.02.2021 Bulletin 2021/07**

(21) Application number: **17760001.2**

(22) Date of filing: **28.02.2017**

(51) Int Cl.:
*A61B 17/58* (2006.01)        *A61L 27/04* (2006.01)
*A61L 27/40* (2006.01)        *A61L 27/30* (2006.01)
*A61L 27/50* (2006.01)        *A61L 31/02* (2006.01)
*A61L 31/08* (2006.01)        *A61L 31/14* (2006.01)
*A61L 31/16* (2006.01)

(86) International application number:
**PCT/JP2017/007807**

(87) International publication number:
**WO 2017/150535 (08.09.2017 Gazette 2017/36)**

(54) **SET SCREW FOR ANTIBACTERIAL DEVICE TO BE IMPLANTED IN VIVO**

STELLSCHRAUBE FÜR ANTIBAKTERIELLE VORRICHTUNG ZUR IN-VIVO-IMPLANTATION

VIS D'ARRÊT ANTIBACTÉRIENNE POUR INSTRUMENT DESTINÉ À ÊTRE IMPLANTÉ DANS UN ORGANISME VIVANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.02.2016 JP 2016038137**

(43) Date of publication of application:
**09.01.2019 Bulletin 2019/02**

(73) Proprietor: **Medtronic Sofamor Danek, Co., Ltd. Osaka-shi, Osaka 553-0003 (JP)**

(72) Inventors:
• **SUGINO, Atsushi**
**Osaka-shi**
**Osaka 553-0003 (JP)**
• **FUKUZAKI, Satoshi**
**Tsu-shi**
**Mie 514-8507 (JP)**

(74) Representative: **FRKelly**
**27 Clyde Road**
**Dublin D04 F838 (IE)**

(56) References cited:
**WO-A1-2015/148800        WO-A2-2007/005842**
**JP-A- H08 506 027        JP-A- 2001 258 894**
**JP-A- 2002 536 048        JP-A- 2013 528 411**
**JP-B2- 5 175 185        US-A1- 2012 123 485**
**US-B1- 8 927 004**

• **KEUN-TAEK OH ET AL: "Electrochemical properties of suprastructures galvanically coupled to a titanium implant", JOURNAL OF BIOMEDICAL MATERIALS RESEARCH PART B: APPLIED BIOMATERIALS, vol. 70B, no. 2, 6 April 2004 (2004-04-06), pages 1-27, XP055624741,**
• **FERRARIS S ET AL: "Antibacterial titanium surfaces for medical implants", MATERIALS SCIENCE AND ENGINEERING C, ELSEVIER SCIENCE S.A, CH, vol. 61, 31 December 2015 (2015-12-31), pages 965-978, XP029403060, ISSN: 0928-4931, DOI: 10.1016/J.MSEC.2015.12.062**

**Description**

TECHNICAL FIELD

The present invention relates to an implant device, and specifically relates to an implant device that achieves an antimicrobial property while it is implanted in vivo for use.

BACKGROUND ART

A variety of instruments such as cages, wires, plates, screws, rods, connectors, crosslinks, hooks, set screws, artificial vertebral bodies, and artificial intervertebral discs are used as orthopedic implant devices. These instruments may be contaminated with microorganisms when they are implanted in vivo for use. If an implant device is colonized by microorganisms, it is very difficult to prevent microbial growth. To prevent microbial growth and infection in the body, it is necessary to perform surgery to remove the implant device for replacement with a new one, which places an enormous burden on the patient. It is desired that an antimicrobial implant device be provided.

As a technique for imparting an antimicrobial property to an implant device, a medical implant system has been proposed (Patent Literature 1) which comprises a metal component containing an antimicrobial metal disposed on an external surface of the implant body; a power source having a first terminal and a second terminal, one of the terminals being in electrical communication with the metal component; and an insulator placed in a current path between the first terminal of the power source and the second terminal of the power source, preventing current flowing from the first terminal from reaching the second terminal without completing a circuit including a conductive body tissue or fluid adjacent to the external surface of the implant system when implanted. Patent Literature 1 discloses that examples of the antimicrobial metal include silver, copper, both silver and copper, both silver and cadmium, and a combination of silver, copper, and cadmium. In Patent Literature 1, the external power source equipment and the terminals are required in addition to the implant body to achieve an antimicrobial property, and a treatment or an external operation is needed to impart an antimicrobial property. Further improvement is required from the viewpoint of reducing the burden on patients.

CITATION LIST

PATENT LITERATURE

PTL 1: Japanese Patent No. 5175185

US 8 927 004 discloses an implant releasing antimicrobial metal ions via galvanic corrosion. The implant comprises a first metal and a second metal. The first metal is selected from silver, zinc or copper; and the second metal is selected from palladium, platinum, gold, molybdenum, titanium, iridium, osmium, niobium and rhenium.

SUMMARY OF INVENTION

TECHNICAL PROBLEM

It is an object of the present invention to provide an implant device that can achieve an antimicrobial property, simply by being implanted in vivo, to reduce the burden on patients.

SOLUTION TO PROBLEM

The present invention is directed to a an antimicrobial set crew as defined in claim 1.

ADVANTAGEOUS EFFECTS OF INVENTION

The implant device of the present invention, once implanted in vivo, can achieve an antimicrobial property without requiring an external operation or treatment, thereby significantly reducing the burden on patients.

BRIEF DESCRIPTION OF DRAWINGS

Fig. 1 is a perspective view of an antimicrobial implant set screw of an embodiment of the present invention.
Fig. 2 is a perspective view of an antimicrobial implant set screw of another embodiment of the present invention.

Fig. 3 is a perspective view of the antimicrobial implant set screw of the present invention shown in Fig. 2.
Fig. 4 is a cross-sectional view of Fig. 3.
Fig. 5 is a cross-sectional view of an embodiment in which a cap head portion includes a porous portion.

DESCRIPTION OF EMBODIMENTS

An antimicrobial implant set screw of the present invention is made of a first material and a second material, wherein at least a surface of the first material comprises an antimicrobial metal; at least a surface of the second material is nobler than the first material; an electric circuit is formed between the first material and the second material when the device is implanted in vivo for use; and the antimicrobial metal ions are eluted from the first material under a presence of an electrolyte to impart an antimicrobial property to a surface and/or a surrounding area of the first material. The antimicrobial implant set screw of the present invention is made of at least two materials, and simply because these two materials are contacted in vivo, a short circuit occurs to form an electric circuit.
The first material is a cobalt-based alloy. More preferably, the cobalt-based alloy comprises cobalt in an amount of 29 to 69 mass%. Still more preferably, the cobalt-based alloy is a cobalt-chromium alloy comprising chromium in an amount of 18 to 30 mass%.
The second material is titanium or a titanium-based alloy. More preferably, the titanium or the titanium-based alloy comprises titanium in an amount of 68 to 100 mass%.
The ratio of an area of the second material to an area of the first material (second material/first material) = 0.2 to 10.8.
When the first material made of the cobalt-based alloy comprising cobalt in an amount of 29 to 69 mass% and the second material made of the titanium-based alloy comprising titanium in an amount of 68 to 100 mass% are used in combination, cobalt ions are eluted from the first material in an amount of 7 nmol/l to 81 $\mu$mol/l to provide a good antimicrobial property.
An antimicrobial implant set screw of the present invention will be described in detail with reference to the attached drawings.

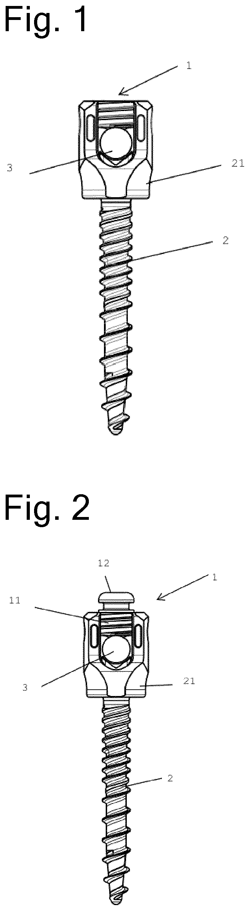

Figs. 1 and 2 are perspective views each showing a state in which the antimicrobial implant set screw of the present invention is used.
An antimicrobial implant set screw 1 is screwed on a head portion 21 of a screw 2 over a rod 3 to hold in place the rod 3 mounted in a seat of the head portion 21 of the screw 2. In the embodiment shown in Fig. 1, the top of the set screw 1 is a flat surface flush with the head portion 21 of the screw 2. In the embodiment shown in Fig. 2, the top of the set screw 1 is projected outside the head portion 21 of the screw 2. The set screws 1 of these embodiments differ in this respect, but otherwise have the same structure.

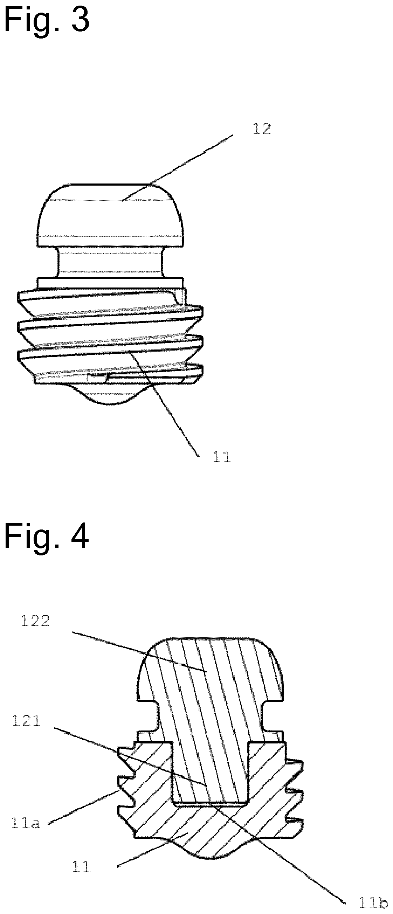

Fig. 3 is a perspective view of the antimicrobial implant set screw shown in Fig. 2, and Fig. 4 is a cross-sectional view of Fig. 3. The antimicrobial implant set screw 1 comprises a set screw body 11 having a screw portion and a head portion wherein the screw portion has a screw groove 11a on the outer surface thereof and the head portion has an opening 11b on the top thereof; and a cap 12 having a leg portion 121 for fitting the opening 11b of the set screw body 11. In the embodiments shown in Figs. 2 to 4, the cap 12 has a head portion 122 that is projected outside the set screw body 11. As shown in Fig. 1, however, the head portion 122 of the cap 12 may not be projected, and may be flush with an upper surface of the set screw body 11. While the antimicrobial implant set screw 1 is implanted in vivo for use, the set screw body 11 and the cap 12 that contain two materials having a potential difference are contacted with each other, which causes an electric circuit to form under the presence of an electrolyte, which allows antimicrobial metal ions to be eluted to create an antimicrobial atmosphere. A first material and a second material may be such that they are contacted with each other under the presence of an electrolyte, while the set screw for an antimicrobial implant device is implanted in vivo for use. For example, preferably, when the set screw body 11 contains the first material, the cap 12 contains the second material. Alternatively, the cap 12 may contain the first material and the second material. For example, the leg portion 121 of the cap 12 may be made of the second material, and the head portion 122 of the cap 12 may be entirely or at least partially made of the first material. An opposite combination is also possible, of course.

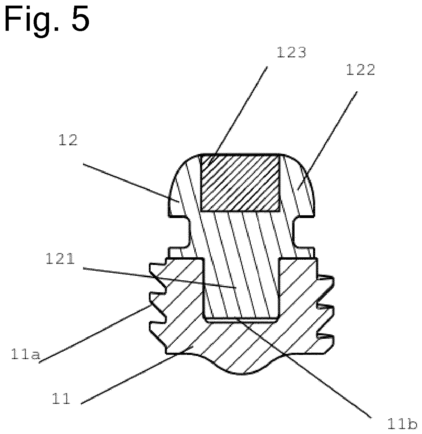

Fig. 5 is a cross-sectional view of an embodiment in which the head portion 122 of the cap 12 of the set screw 1 includes a porous portion 123. The porous portion may be a porous region composed of intertwined or agglutinated fibers comprising the first material or the second material, or may be a porous structure in which the entire head portion 122 of the cap 12 is such a porous region. For example, preferably, when the porous portion 123 that contains the first material is a part of the head portion 122, the remaining portion of the head portion 122 is made of the second material. For example, when the entire head portion 122 is a porous structure containing the first material, the leg portion 121 may be made of the second material. Alternatively, for example, the entire cap 12 including the porous portion 123 may be made of the first material, and the screw body 11 may be made of the second material. In any of these embodiments, the combination of the first material and the second material is not limited to that described above, and an opposite combination is also possible.

Alternatively, the cap 12 may include a porous region in which fibers containing the first material and fibers containing the second material are mixed and intertwined or agglutinated, or may be made of a porous structure in which fibers containing the first material and fibers containing the second material are mixed and intertwined or agglutinated. Because both the first material and the second material have a fibrous shape, the surface areas of the materials increase to increase the contact area between the first material and the second material. Furthermore, because the porous structures are adopted, the amount of antibacterial metal ions eluted can be increased, and the elution of the ions can be promoted.

Furthermore, the portion 123 of the head portion 122 or the entire head portion 122 of the cap 12 may be made of a sintered body formed by sintering powder particles of the first material or the second material. When the portion 123 of the head portion 122 is made of the sintered body, the remaining portion of the head portion 122 may be made of a solid portion of the same material or a different material. To form the sintered portion and the solid portion, compression molding, sintering, diffusion bonding, additive manufacturing using an electron beam or laser, metal injection molding, spark plasma sintering, or a combination of these methods can be suitably used.

The second material is preferred as the material that forms the porous portion, because the surface area of the second material can be increased to increase the ratio of the surface area of the second material to the surface area of the first material, resulting in an increased amount of antibacterial metal ions eluted from the first material.

EXAMPLES

The present invention will be hereinafter described in detail with examples; however, the invention is not limited to these examples.

[Example 1]

(Preparation of Samples)

A commercially available biocompatible titanium alloy (titanium content: 88 to 91 mass%) and a commercially available biocompatible cobalt-chromium alloy (cobalt content: 58 to 69 mass%, chromium content: 26 to 30 mass%) were processed into 3-mm-thick coin-shaped samples (only the titanium alloy of Sample No. 3 was processed into a flat plate-shaped sample) having the various areas shown in Table 1, and these samples were laminated in the various combinations shown in Table 1 and shorted to each other. As comparative samples, a 1-mm-thick flat plate-shaped polyethylene sample (control), a 3-mm-thick coin-shaped sample made of the cobalt-chromium alloy (Comparison 1), and a 3-mm-thick coin-shaped sample made of the titanium alloy (Comparison 2) were similarly prepared.

[Table 1]

Table 1 Sample Conditions

| Sample No. | Area Ratio* | Surface Area/mm$^2$ | |
| --- | --- | --- | --- |
| | | Titanium Alloy | Cobalt-Chromium Alloy |
| 1 | 1.0 | 518 | 518 |
| 2 | 3.3 | 1696 | 518 |
| 3 | 10.8 | 5600 | 518 |
| 4 | 0.2 | 103 | 518 |
| Comparison 1 | - | - | 518 |
| Comparison 2 | - | 518 | - |
| Control | - | Polyethylene | |
| * Area Ratio = Surface Area of Titanium Alloy/Surface Area of Cobalt-Chromium Alloy | | | |

(Antimicrobial Property Test)

An antimicrobial property was evaluated with reference to JIS Z 2801: Antibacterial products - Test for antibacterial activity and efficacy.

Each of the samples shown in Table 1 was placed in a petri dish, and 40 μl of *Staphylococcus aureus* (NBRC 12732) suspended in a nutrient broth (1/100) was added dropwise. A polyethylene film was placed over the test bacterial suspension added. The suspension was cultured at 35°C for 24 hours, and then cells were washed out from the sample. The resulting wash-out suspension was serially diluted in a 10-fold dilution series and cultured at 35°C for 24 hours using the agar plate culture method, and the number of viable cells was calculated from the number of colony forming units (CFUs) formed. The difference between the logarithmic value of the number of viable cells on the polyethylene sample as a control after 24-hour contact (Nc) and the logarithmic value of the number of viable cells on each sample (N) was determined as the antimicrobial activity value.

[Math. 1]

$$\text{Antimicrobial activity value} = \log(Nc/N)$$

Table 2 shows the number of viable cells and the antimicrobial activity value for each sample. A sample having an antimicrobial activity value of 2.0 or more was determined to have antimicrobial efficacy. The number of viable cells decreased (the antimicrobial activity value increased) as the area ratio (the surface area of the sample made of the titanium alloy/the surface area of the sample made of the cobalt-chromium alloy) increased.

[Table 2]

Table 2 Results of Antimicrobial Property Test

| Sample | Number of Viable Cells/CFU/Coupon | Antimicrobial Activity Value |
|---|---|---|
| Control: Polyethylene | $1.1 \times 10^6$ | - |
| 1 | $6.8 \times 10^3$ | 2.21 |
| 2 | $2.4 \times 10^3$ | 2.66 |
| 3 | $4.0 \times 10^2$ | 3.44 |
| Comparison 1 | $9.2 \times 10^3$ | 2.08 |

[Example 2]

(Preparation of Samples)

An implant device made of the titanium alloy and the cobalt-chromium alloy (the area ratio of the titanium alloy to the cobalt-chromium alloy was set to 0.89) was prepared (implant device 1). As a comparative example, an implant device made of only the titanium alloy having completely the same shape was similarly prepared (implant device 2).

[Table 3]

Table 3 Composition of Implant Device

| Implant Device No. | Area Ratio* | Combination of Materials | |
|---|---|---|---|
| | | Titanium Alloy | Cobalt-Chromium Alloy |
| 1 | 0.89 | ○ | ○ |
| 2 | --- | ○ | |
| * Area Ratio = Surface Area of Member Made of Titanium Alloy/Surface Area of Member Made of Cobalt-Chromium Alloy | | | |

(Antimicrobial Property Test)

Each of the implant devices was placed in a polypropylene tube, and immersed in 1 ml of *Staphylococcus aureus* (NBRC 12732) suspended in a nutrient broth (1/100). After 24 hours of culture at 35°C, the culture medium was collected. The collected culture medium was serially diluted in a 10-fold dilution series and cultured at 35°C for 24 hours using the agar plate culture method, and the number of viable cells was determined from the number of colony forming units (CFUs) formed.

Table 4 shows the number of viable cells and the antimicrobial activity value for each implant device. In the implant device 1 in which the area ratio of the titanium alloy to the cobalt-chromium alloy was appropriately set, the number of viable cells significantly decreased. In contrast, in the implant device 2 made of the titanium alloy only, a decrease in the number of viable cells was not observed.

[Table 4]

Table 4 Results of Antimicrobial Property Test for Implant Device

| Implant Device No. | Number of Viable Cells/CFU/Coupon | Antimicrobial Activity Value |
| --- | --- | --- |
| Polyethylene | $7.6 \times 10^7$ | - |
| 1 | $1.2 \times 10^5$ | 2.80 |
| 2 | $7.0 \times 10^7$ | 0.04 |

**Claims**

1.  An antimicrobial set screw for an antimicrobial implant device comprising a set screw body having a screw portion and a head portion wherein the screw portion has a screw groove on the outer surface thereof and the head portion has an opening on the top thereof; and a cap having a leg portion for fitting the opening of the set screw body, wherein at least a surface of the opening of the set screw body and at least a surface of the leg portion of the cap comprise a first material and a second material that have a potential difference;
    the first material comprises an antimicrobial metal;
    the second material is nobler than the first material;
    an electric circuit is formed between the first material and the second material when the set screw is implanted in vivo for use;
    ions of the antimicrobial metal are eluted from the first material under a presence of an electrolyte to impart an antimicrobial property to a surface and/or a surrounding area of the first material;
    wherein the first material is a cobalt-based alloy;
    wherein the second material is titanium or a titanium-based alloy; and
    wherein a ratio of a surface area of the second material to a surface area of the first material (second material/first material) is in a range of 0.2 to 10.8.

2.  The antimicrobial set screw for an antimicrobial implant device according to claim 1, wherein the cobalt-based alloy comprises cobalt in an amount of 29 to 69 mass%.

3.  The antimicrobial set screw for an antimicrobial implant device according to claim 1 or 2, wherein the cobalt-based alloy is a cobalt-chromium alloy.

4.  The antimicrobial set screw for an antimicrobial implant device according to claim 3, wherein the cobalt-chromium alloy comprises chromium in an amount of 18 to 30 mass%.

5.  The antimicrobial set screw for an antimicrobial implant device according to claim 1, wherein the titanium or the titanium-based alloy comprises titanium in an amount of 68 to 100 mass%.

6.  The antimicrobial set screw for an antimicrobial implant device according to any one of claims 1 to 5, wherein
    the set screw body has a porous portion comprising the first material or the second material;
    the cap has a porous portion comprising the first material or the second material; and the porous portion of the set screw body and the porous portion of the cap comprise different materials with each other.

7. The antimicrobial set screw for an antimicrobial implant device according to claim 6, wherein the porous portion comprises a porous region composed of intertwined or agglutinated fibers comprising the first material or the second material.

8. The antimicrobial set screw for an antimicrobial implant device according to claim 6, wherein the porous portion is a porous structure composed of intertwined or agglutinated fibers comprising the first material or the second material.

9. The antimicrobial set screw for an antimicrobial implant device according to claim 6, wherein the porous portion has a sintered body formed by sintering powder particles of the first material or the second material.

10. The antimicrobial set screw for an antimicrobial implant device according to any one of claims 1 to 9, wherein the cap is fit on the set screw body and projected outside the set screw body.


**Patentansprüche**

1. Antimikrobielle Stellschraube für eine antimikrobielle Implantatvorrichtung, die Folgendes umfasst: einen Stellschraubenkörper, der einen Schraubenabschnitt und einen Kopfabschnitt aufweist, wobei der Schraubenabschnitt eine Schraubennut an der Außenoberfläche davon aufweist und der Kopfabschnitt eine Öffnung an der Oberseite davon aufweist; und eine Kappe, die einen Fußabschnitt zum Befestigen der Öffnung des Stellschraubenkörpers aufweist, wobei
wenigstens eine Oberfläche der Öffnung des Stellschraubenkörpers und wenigstens eine Oberfläche des Fußabschnitts der Kappe ein erstes Material und ein zweites Material umfassen, die eine Potentialdifferenz aufweisen;
das erste Material ein antimikrobielles Metall umfasst;
das zweite Material edler als das erste Material ist;
ein elektrischer Stromkreis zwischen dem ersten Material und dem zweiten Material ausgebildet ist, wenn die Stellschraube zur Verwendung in vivo implantiert wird;
Ionen des antimikrobiellen Metalls aus dem ersten Material unter einer Anwesenheit eines Elektrolyten eluiert werden, um einer Oberfläche und/oder einem Umgebungsbereich des ersten Materials eine antimikrobielle Eigenschaft zu verleihen; wobei das erste Material eine Legierung auf Kobaltbasis ist;
wobei das zweite Material Titan oder eine Legierung auf Titanbasis ist; und
wobei ein Verhältnis eines Oberflächenbereichs des zweiten Materials zu einem Oberflächenbereich des ersten Materials (zweites Material/erstes Material) in einem Bereich von 0,2 bis 10,8 liegt.

2. Antimikrobielle Stellschraube für eine antimikrobielle Implantatvorrichtung nach Anspruch 1, wobei die Legierung auf Kobaltbasis Kobalt in einer Menge von 29 bis 69 Massen-% umfasst.

3. Antimikrobielle Stellschraube für eine antimikrobielle Implantatvorrichtung nach Anspruch 1 oder 2, wobei die Legierung auf Kobaltbasis eine Kobalt-Chrom-Legierung ist.

4. Antimikrobielle Stellschraube für eine antimikrobielle Implantatvorrichtung nach Anspruch 3, wobei die Kobalt-Chrom-Legierung Chrom in einer Menge von 18 bis 30 Massen-% umfasst.

5. Antimikrobielle Stellschraube für eine antimikrobielle Implantatvorrichtung nach Anspruch 1, wobei das Titan oder die Legierung auf Titanbasis Titan in einer Menge von 68 bis 100 Massen-% umfasst.

6. Antimikrobielle Stellschraube für eine antimikrobielle Implantatvorrichtung nach einem der Ansprüche 1 bis 5, wobei der Stellschraubenkörper einen porösen Abschnitt aufweist, der das erste Material oder das zweite Material umfasst;
die Kappe einen porösen Abschnitt aufweist, der das erste Material oder das zweite Material umfasst; und
der poröse Abschnitt des Stellschraubenkörpers und der poröse Abschnitt der Kappe unterschiedliche Materialien miteinander umfassen.

7. Antimikrobielle Stellschraube für eine antimikrobielle Implantatvorrichtung nach Anspruch 6, wobei der poröse Abschnitt eine poröse Region umfasst, die aus verwebten oder agglutinierten Fasern besteht, die das erste Material oder das zweite Material umfassen.

**8.** Antimikrobielle Stellschraube für eine antimikrobielle Implantatvorrichtung nach Anspruch 6, wobei der poröse Abschnitt eine poröse Struktur ist, die aus verwebten oder agglutinierten Fasern besteht, die das erste Material oder das zweite Material umfassen.

**9.** Antimikrobielle Stellschraube für eine antimikrobielle Implantatvorrichtung nach Anspruch 6, wobei der poröse Abschnitt einen gesinterten Körper aufweist, der durch Sintern von Pulverpartikeln des ersten Materials oder des zweiten Materials ausgebildet ist.

**10.** Antimikrobielle Stellschraube für eine antimikrobielle Implantatvorrichtung nach einem der Ansprüche 1 bis 9, wobei die Kappe auf dem Stellschraubenkörper befestigt ist und außerhalb des Stellschraubenkörpers vorsteht.

**Revendications**

**1.** Vis de pression antimicrobienne pour un dispositif d'implant antimicrobien comprenant un corps de vis de pression ayant une partie de vis et une partie de tête, la partie de vis ayant une rainure de vis sur sa surface extérieure et la partie de tête ayant une ouverture sur son dessus ; et un capuchon ayant une partie de patte pour s'adapter à l'ouverture du corps de vis de pression,
au moins une surface de l'ouverture du corps de vis de pression et au moins une surface de la partie de patte du capuchon comprenant un premier matériau et un second matériau qui ont une différence de potentiel ;
le premier matériau comprenant un métal antimicrobien ;
le second matériau étant plus noble que le premier matériau ;
un circuit électrique étant formé entre le premier matériau et le second matériau lorsque la vis de pression est implantée in vivo pour utilisation ;
des ions du métal antimicrobien étant élués du premier matériau en présence d'un électrolyte pour conférer une propriété antimicrobienne à une surface et/ou à une zone environnante du premier matériau ;
le premier matériau étant un alliage à base de cobalt ;
le second matériau étant du titane ou un alliage à base de titane ; et
un rapport d'une superficie du second matériau à une superficie du premier matériau (second matériau/premier matériau) étant compris entre 0,2 et 10,8.

**2.** Vis de pression antimicrobienne pour un dispositif d'implant antimicrobien selon la revendication 1, l'alliage à base de cobalt comprenant du cobalt en une quantité de 29 à 69 % en masse.

**3.** Vis de pression antimicrobienne pour un dispositif d'implant antimicrobien selon la revendication 1 ou 2, l'alliage à base de cobalt étant un alliage de cobalt-chrome.

**4.** Vis de pression antimicrobienne pour un dispositif d'implant antimicrobien selon la revendication 3, l'alliage cobalt-chrome comprenant du chrome en une quantité de 18 à 30 % en masse.

**5.** Vis de pression antimicrobienne pour un dispositif d'implant antimicrobien selon la revendication 1, le titane ou l'alliage à base de titane comprenant du titane en une quantité de 68 à 100 % en masse.

**6.** Vis de pression antimicrobienne pour un dispositif d'implant antimicrobien selon l'une quelconque des revendications 1 à 5,
le corps de vis de pression ayant une partie poreuse comprenant le premier matériau ou le second matériau ;
le capuchon ayant une partie poreuse comprenant le premier matériau ou le second matériau ; et
la partie poreuse du corps de vis de pression et la partie poreuse du capuchon comprenant des matériaux différents les uns des autres.

**7.** Vis de pression antimicrobienne pour un dispositif d'implant antimicrobien selon la revendication 6, la partie poreuse comprenant une région poreuse composée de fibres entrelacées ou agglutinées comprenant le premier matériau ou le second matériau.

**8.** Vis de pression antimicrobienne pour un dispositif d'implant antimicrobien selon la revendication 6, la partie poreuse étant une structure poreuse composée de fibres entrelacées ou agglutinées comprenant le premier matériau ou le second matériau.

**9.** Vis de pression antimicrobienne pour un dispositif d'implant antimicrobien selon la revendication 6, la partie poreuse ayant un corps fritté formé par frittage de particules de poudre du premier matériau ou du second matériau.

**10.** Vis de pression antimicrobienne pour un dispositif d'implant antimicrobien selon l'une quelconque des revendications 1 à 9, le capuchon étant ajusté sur le corps de la vis de pression et faisant saillie à l'extérieur du corps de la vis de pression.

# Fig. 1

# Fig. 2

Fig. 3

Fig. 4

# Fig. 5

**EP 3 424 450 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5175185 B

- US 8927004 B